# EUROPEAN PATENT APPLICATION

(11) **EP 4 656 205 A1**
(43) Date of publication of application: **03.12.2025**
(21) Application number: 24747328.3
(22) Date of filing: 24.01.2024
(51) Int. Cl.: A61K 39/395, A61K 31/475, A61K 31/573, A61K 31/675, A61K 31/704, A61K 45/00, A61P 35/00, A61P 43/00, C07K 16/30

(54) **METHOD FOR TREATING CANINE B CELL LYMPHOMA**

(30) Priority: 25.01.2023 JP 2023009500
(71) Applicant: Nippon Zenyaku Kogyo Co., Ltd., Koriyama-shi, Fukushima 963-0196 (JP)
(72) Inventor: MIZUNO Takuya, Yamaguchi-shi, Yamaguchi 753-8515 (JP)
(74) Representative: Potter Clarkson
(86) International application number: PCT/JP2024/001991
(87) International publication number: WO 2024/158001

(57) **Abstract**

Provided is a method for treating canine B-cell lymphoma that is more effective than conventional methods. The method for treating canine B-cell lymphoma includes administering an anti-canine CD20 monoclonal antibody in combination with a chemotherapeutic agent, simultaneously or sequentially. The chemotherapeutic agent is one or more of vincristine, cyclophosphamide, prednisolone, and doxorubicin. The monoclonal antibody against canine CD20 and the chemotherapeutic agent are administered in combination, simultaneously or sequentially.

## Description

### TECHNICAL FIELD

The present invention relates to a method for treating canine B-cell lymphoma. More particularly, the present invention relates to a method for treating canine B-cell lymphoma that is more effective than conventional treatment methods.

### BACKGROUND ART

Various methods such as chemotherapy, radiation therapy, and immunotherapy have been known for the treatment of canine B-cell lymphoma. As chemotherapy, CHOP chemotherapy or the like in which a plurality of anticancer agents are administered in combination has been performed. However, the survival time (median value) after starting the treatment is as short as about one year, and in most cases, the disease relapses after remission. Accordingly, the CHOP chemotherapy was not a sufficient treatment (Non-Patent Documents 1 and 2). In recent years, attempts have been made to develop an antibody therapeutic targeting CD20 for use in the treatment.

For example, Patent Documents 1 and 2 disclose antibodies that recognize canine or feline CD20 and monoclonal antibodies as binding agents that bind to canine CD20, and suggest use of chemotherapeutic agents such as cyclophosphamide, doxorubicin, vincristine, and prednisone in combination with the antibodies.

In addition, Patent Document 3 discloses monoclonal antibodies against extracellular regions of canine CD20, and suggests to administer cyclophosphamide or fludarabine in advance to reduce T cells in the body of a dog, and then treat cancer such as lymphoma in a method for treating canine B-cell lymphoma.

As described above, in the treatment of canine B-cell lymphoma, it has been suggested to use an antibody therapeutic targeting CD20 in combination with a chemotherapeutic agent, but there have been no examples in which specific effects were confirmed, and the type, combination, dosage, and the like of the chemotherapeutic agent have not even been studied (Non-Patent Document 3). Further, because the effect of the antibody therapeutic targeting CD20 varies depending on the antibody, it could not be said that a simple combination of the antibody therapeutic and the chemotherapeutic agent described above is effective in the treatment of canine B-cell lymphoma.

Therefore, the present inventors selected monoclonal antibodies against canine CD20 and chemotherapeutic agents that are useful for the treatment of canine B-cell lymphoma, and by administering them in combination, attempted to provide a method for treating canine B-cell lymphoma that is more effective than conventional treatment methods.

### CITATION LIST

### PATENT LITERATURE

Patent Document 1: JP 2013-520990 A
Patent Document 2: JP 2014-532649 A
Patent Document 3: JP 2016-013104 A

### NON PATENT LITERATURE

Non-Patent Document 1: Zandvliet M. Canine lymphoma: a review. Vet Q. 2016 Jun; 36(2): 76-104
Non-Patent Document 2: Garrett LD, Thamm DH, Chun R, Dudley R, Vail DM. Evaluation of a 6-month chemotherapy protocol with no maintenance therapy for dogs with lymphoma. J Vet Intern Med. 2002 Nov-Dec; 16(6): 704-9
Non-Patent Document 3: Atherton MJ, Mason NJ. Bite-size introduction to canine hematologic malignancies. Blood Adv. 2022 Jul 12; 6(13): 4073-4084
Non-Patent Document 4: David M. Vail, Douglas H. Thamm, Julias M. Liptak, 33 - Hematopoietic Tumors, Editor(s): David M. Vail, Douglas H. Thamm, Julias M. Liptak, Withrow and MacEwen's Small Animal Clinical Oncology (Sixth Edition), W.B. Saunders, 2019, Pages 688-772, ISBN 9780323594967

### SUMMARY OF INVENTION

### TECHNICAL PROBLEM

An object of the present invention is to provide a method for treating canine B-cell lymphoma that is more effective than conventional methods.

### SOLUTION TO PROBLEM

As a result of intensive studies to solve the problems of the present invention, the present inventors found that administration of an anti-canine CD20 monoclonal antibody and a chemotherapeutic agent that are useful for the treatment of canine B-cell lymphoma in combination, either simultaneously or sequentially, can provide a method for treating canine B-cell lymphoma that is more effective than conventional methods. Thereby, the present invention was completed.

According to the method for treating canine B-cell lymphoma of the present invention, relapse of B-cell lymphoma is suppressed even in the period with risk of relapse after completion of the administration, and survival of dogs can be maintained for a long period of time exceeding one year.

That is, the present invention relates to a method for treating canine B-cell lymphoma recited in the following (1) to (9).
(1) A method for treating canine B-cell lymphoma comprising administering an anti-canine CD20 monoclonal antibody in combination with a chemotherapeutic agent, simultaneously or sequentially, wherein the antibody comprises a heavy chain variable region and a light chain variable region, wherein the heavy chain variable region comprises V_{H} CDR1 of GFTFNDYWMS (SEQ ID NO: 6), V_{H} CDR2 of DIKYDGSYTNYAPSLKN (SEQ ID NO: 7), and V_{H} CDR3 of EAYYYSGDY (SEQ ID NO: 8), and wherein the light chain variable region comprises V_{L} CDR1 of KTNQNVDYYGNSYMH (SEQ ID NO: 9), V_{L} CDR2 of LASNLAS (SEQ ID NO: 10), and V_{L} CDR3 of QQSRNLPYT (SEQ ID NO: 11).
(2) The method as recited in (1), wherein the chemotherapeutic agent is one or more, two or more, three or more, or all of vincristine, cyclophosphamide, prednisolone, and doxorubicin.
(3) The method as recited in (1) or (2), wherein the B-cell lymphoma is canine multicentric large B-cell lymphoma.
(4) An anti-canine CD20 monoclonal antibody for use in a method of treating canine B-cell lymphoma, wherein the method comprises administering said antibody in combination with a chemotherapeutic agent, simultaneously or sequentially, wherein the antibody comprises a heavy chain variable region and a light chain variable region, wherein the heavy chain variable region comprises V_{H} CDR1 of GFTFNDYWMS (SEQ ID NO: 6), V_{H} CDR2 of DIKYDGSYTNYAPSLKN (SEQ ID NO: 7), and V_{H} CDR3 of EAYYYSGDY (SEQ ID NO: 8), and wherein the light chain variable region comprises V_{L} CDR1 of KTNQNVDYYGNSYMH (SEQ ID NO: 9), V_{L} CDR2 of LASNLAS (SEQ ID NO: 10), and V_{L} CDR3 of QQSRNLPYT (SEQ ID NO: 11).
(5) The anti-canine CD20 monoclonal antibody as recited in (4), wherein the chemotherapeutic agent is one or more, two or more, three or more, or all of vincristine, cyclophosphamide, prednisolone, and doxorubicin.
(6) The anti-canine CD20 monoclonal antibody as recited in (4) or (5), wherein the B-cell lymphoma is canine multicentric large B-cell lymphoma.
(7) A kit comprising an anti-canine CD20 monoclonal antibody and a chemotherapeutic agent for use in a method of treating canine B-cell lymphoma, wherein the antibody comprises a heavy chain variable region and a light chain variable region, wherein the heavy chain variable region includes V_{H} CDR1 of GFTFNDYWMS (SEQ ID NO: 6), V_{H} CDR2 of DIKYDGSYTNYAPSLKN (SEQ ID NO: 7), and V_{H} CDR3 of EAYYYSGDY (SEQ ID NO: 8), and wherein the light chain variable region includes V_{L} CDR1 of KTNQNVDYYGNSYMH (SEQ ID NO: 9), V_{L} CDR2 of LASNLAS (SEQ ID NO: 10), and V_{L} CDR3 of QQSRNLPYT (SEQ ID NO: 11).
(8) The kit as recited in (7), wherein the chemotherapeutic agent is one or more, two or more, three or more, or all of vincristine, cyclophosphamide, prednisolone, and doxorubicin.
(9) The kit as recited in (7) or (8), wherein the B-cell lymphoma is canine multicentric large B-cell lymphoma.

### ADVANTAGEOUS EFFECTS OF INVENTION

According to the present invention, it is possible to provide a method for treating canine B-cell lymphoma that is more effective than conventional methods. In addition, by providing a kit for treating canine B-cell lymphoma including an anti-canine CD20 monoclonal antibody and a chemotherapeutic agent, it is also possible to treat canine B-cell lymphoma more conveniently.

### BRIEF DESCRIPTION OF DRAWINGS

[Fig. 1]

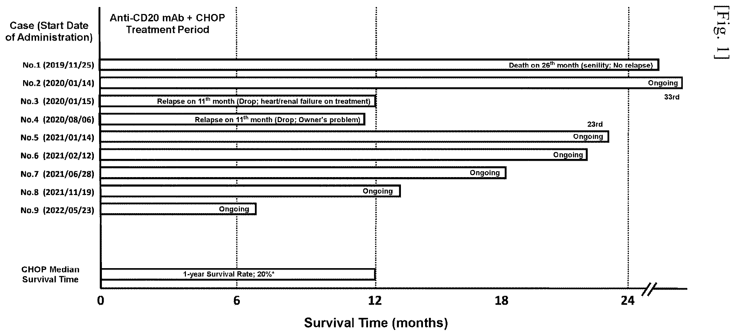

   Fig. 1 is a graph showing survival times of each treatment subject (Example 1).
[Fig. 2]

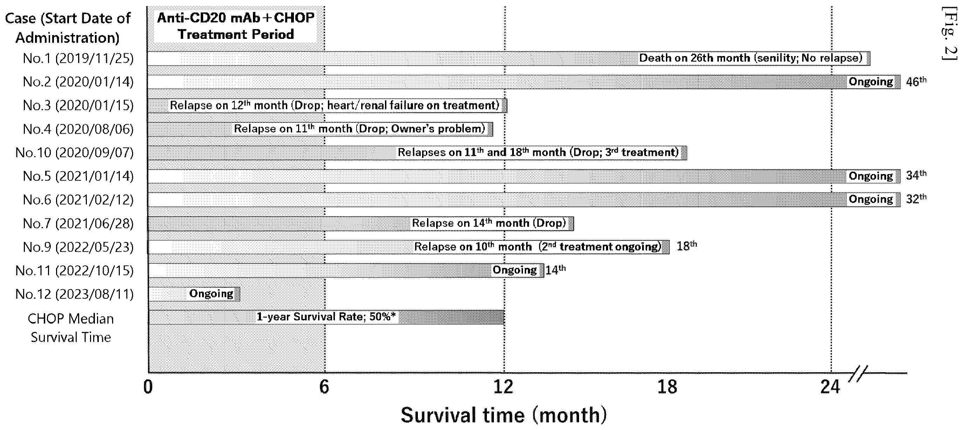

   Fig. 2 is a graph showing survival times of each treatment subject (Example 1).

### DESCRIPTION OF EMBODIMENTS

The "method for treating canine B-cell lymphoma" of the present invention refers to a method including a step of "simultaneously or sequentially administering a combination of" "an anti-canine CD20 monoclonal antibody" and "a chemotherapeutic agent" to a dog to be treated.

The term "simultaneously" used in the present invention includes not only a case where the administration is started at the same time, but also a case where the administration is performed in the same time period on the same day. Examples of the time period include early morning, morning, afternoon, evening, and night. In addition, the term "sequentially" means that agents are administered within a certain time frame in which the agents can therapeutically act, and includes cases where the agents are administered in different time periods on the same day or on different days within a certain period such as one week as long as the agents can therapeutically act.

Examples of the step of "simultaneously or sequentially administering a combination of" the "anti-canine CD20 monoclonal antibody" and the "chemotherapeutic agent" according to invention of the present application include: when the treatment period is 5 weeks, administration in the first week of a combination of two or more specific "chemotherapeutic agents"; administration in the second week of a combination of two or more "chemotherapeutic agents" different from those administered in the first week; administration in the third week of a combination of the "anti-canine CD20 monoclonal antibody" and the "chemotherapeutic agent"; administration in the fourth week of a combination of two or more "chemotherapeutic agents" different from those administered in the first and the second weeks; and withdrawal in the fifth week.

The "method for treating canine B-cell lymphoma" of the present invention may further include, in addition to the step of "administering an anti-canine CD20 monoclonal antibody in combination with a chemotherapeutic agent, simultaneously or sequentially," other steps useful for treating canine B-cell lymphoma.

The "canine B-cell lymphoma" to be treated by the present invention may be any types, and examples thereof include multicentric large B-cell lymphoma, mediastinal (thymic) lymphoma, gastrointestinal lymphoma, cutaneous lymphoma, and extranodal lymphoma. The multicentric large B-cell lymphoma particularly includes diffuse large B-cell lymphoma (DLBCL).

The "anti-canine CD20 monoclonal antibody" of the present invention refers to an antibody that recognizes a canine CD20 molecule. The "anti-canine CD20 monoclonal antibody" of the present invention is an antibody that can recognize the canine CD20 molecule and can be used for the treatment of canine B-cell lymphoma. It may be a commercially available antibody or an antibody produced in-house by a conventionally known method or the like. Furthermore, an antibody that has been subjected to a treatment such as defucosylation may also be used.

The "anti-canine CD20 monoclonal antibody" of the present invention may be an agent in any form as long as it is effective for the treatment of canine B-cell lymphoma, and may be provided as a liquid for intravenous injection or drip infusion, powder, tablet, capsule, or the like. In addition, other components, drugs, pharmaceutically acceptable additives, and the like may be contained.

The "chemotherapeutic agent" of the present invention refers to a "chemotherapeutic agent" that can be used for the treatment of canine B-cell lymphoma, and may be a commercially available agent or an agent produced in-house by a conventionally known method or the like.

The "chemotherapeutic agent" of the present invention may be an agent in any form as long as it is effective for the treatment of canine B-cell lymphoma, and may be provided as a liquid for intravenous injection or drip infusion, powder, tablet, capsule, or the like. In addition, other components, drugs, pharmaceutically acceptable additives, and the like may be contained.

Examples of such a "chemotherapeutic agent" include vincristine, cyclophosphamide, prednisolone, prednisone, and doxorubicin. These "chemotherapeutic agents" may be commercially available products or agents originally prepared. Examples of the commercially available products include Oncovin for Injection 1 mg (manufactured by Nippon Kayaku Co., Ltd.) for vincristine, and Prednisolone Tablets "Takeda" 5 mg (manufactured by Teva Takeda Pharma Ltd.) for prednisolone. Examples of doxorubicin include Adriacin Injection 10 and Adriacin Injection 50 (both manufactured by Sandoz Pharma K.K.), and Doxorubicin Hydrochloride for Injection 10 mg "NK" or Doxorubicin Hydrochloride for Injection 50 mg "NK" (both manufactured by Nippon Kayaku Co., Ltd.), which are doxorubicin hydrochlorides, may also be used.

In the treatment of canine B-cell lymphoma, these agents may be used alone, but it is more preferable to use two or more, three or more, or four or more of them in combination.

In treating canine B-cell lymphoma, the "anti-canine CD20 monoclonal antibody" and the "chemotherapeutic agent" of the present invention can be administered to a treatment subject by, for example, intravenous injection, intramuscular injection, subcutaneous injection, drip infusion, or oral administration.

In this case, the "anti-canine CD20 monoclonal antibody" of the present invention is preferably administered at a dose of 0.1 to 25 mg/kg, once or at an interval of about once every 4 weeks. Furthermore, the antibody may be administered at a dose of 0.5 mg to 25 mg/kg, once or at an interval of about once every 2 to 4 weeks.

Among the "chemotherapeutic agents," vincristine is preferably administered at an interval of about once every 2 weeks at a dose of 0.5 to 0.75 mg/m², and cyclophosphamide is preferably administered at an interval of about once every 4 weeks at a dose of 200 to 250 mg/m².

In addition, prednisolone is preferably administered daily at a dose within the range of 0.5 to 2 mg/kg (10 to 30 mg/m² in a large dog over 30 kg). The timing of administration and the presence or absence of administration may be adjusted as necessary.

Furthermore, doxorubicin is preferably administered at an interval of about once every 3 to 4 weeks at a dose of about 1 mg/kg when the body weight of the treatment subject is less than 10 kg, and at a dose within the range of 20 to 30 mg/m² when the body weight of the treatment subject is 10 kg or more. When doxorubicin cannot be used due to the decreased cardiac contractility of the treatment subject, mitoxantrone (trade name: Novantrone) (5 to 6 mg/m²) may be administered as an alternative.

The "kit for treating canine B-cell lymphoma" of the present invention is a kit including the "anti-canine CD20 monoclonal antibody" and the "chemotherapeutic agent" of the present invention as components, and may further contain other components such as an injection device and an infusion pump/syringe pump.

Hereinafter, Examples or the like of the present invention will be described, but the present invention is not limited thereto.

### EXAMPLES

### 1. Preparation of Sample

### 1) Anti-canine CD20 monoclonal antibody (hereinafter, may simply be referred to as "anti-CD20 antibody")

As an anti-canine CD20 monoclonal antibody, 4E1-7-B_f antibody was produced in the same manner as described in Reference Document 2 (JP 2021-059499 A).

That is, a heavy chain variable region consisting of the amino acid sequence represented by SEQ ID NO: 1 and a light chain variable region consisting of the amino acid sequence represented by SEQ ID NO: 2 as variable regions (mouse), and a light chain constant region consisting of the amino acid sequence represented by SEQ ID NO: 3 and a heavy chain constant region consisting of the amino acid sequence represented by SEQ ID NO: 4 as constant regions (dog, isotype B = H chain, kappa = L chain) were constructed by overlap PCR, and ligated to a pCAGGS-MCS vector to obtain an expression plasmid pCAGGS-4E1-7VH-CHB#31. The obtained plasmid was introduced into BINDS-09 cell line using ExpiCHO (trade mark) expression system (Thermo Fisher Scientific Inc.: A29133). The BINDS-09 cell line was obtained by knocking out a fucose transferase (FUT8) gene by genome editing (crisper/cas9) using a CHO cell (Thermo Fisher Scientific Inc.: ExpiCHO-S (trade mark)), and was provided by Kato Laboratory, Tohoku University.

After the plasmid introduction, the cells were cultured using a system for the High titer of the same expression system, and the culture supernatant was collected and purified with Ab Capcher (trade mark) column (ProteNova Co., Ltd.) to obtain the 4E1-7-B_f antibody.

### 2) Chemotherapeutic Agent

(1) Vincristine (Oncovin for Injection 1 mg, Nippon Kayaku Co., Ltd.)
(2) Cyclophosphamide (Endoxan Powder for Oral Use 100mg, Shionogi & Co., Ltd.)
(3) Prednisolone (Prednisolone Tablets "Takeda" 5 mg, Teva Takeda Pharma Ltd.)
(4) Doxorubicin (Adriacin Injection 10, Sandoz Pharma K.K.)

### 2. Method for Treating Canine B-cell Lymphoma

Dogs suffering from B-cell lymphoma were treated for B-cell lymphoma by administering a combination of the anti-CD20 antibody and each of the chemotherapeutic agents, either simultaneously or sequentially.

The combination, dosage and dosing schedule of the anti-CD20 antibody and each of the chemotherapeutic agents were adjusted according to the treatment subject.

### [Example 1]

In the Animal Medical Center of Yamaguchi University, dogs diagnosed as suffering from multicentric large B-cell lymphoma by cytologic diagnosis or histopathological examination and having no history of chemotherapy, radiotherapy, or various immunotherapies were treated by the method of 2. described above. The dog breed, starting age, sex and the like of each treatment subject are shown in Table 1.

For the treatment, the anti-CD20 antibody and each of the chemotherapeutic agents described in the above 1 were prepared with saline to obtain the following dosages. Thereafter, according to the administration protocol shown in Table 2, each drug was administered to each treatment subject by intravenous infusion.

For each treatment subject, T cells and B cells contained in the peripheral blood at the start of administration and after the start of administration were examined to confirm the remission maintenance effect and the survival benefit in the treatment of B-cell lymphoma.

### Preparation of Each Agent

### 1) Anti-canine CD20 Monoclonal Antibody

The antibody was administered by intravenous injection at a dose of 5 mg per 1 kg of body weight of the treatment subject.

### 2) Chemotherapeutic Agent

### (1) Vincristine

Vincristine was administered to the treatment subject by intravenous injection at a dose of 0.7 mg/m².

### (2) Cyclophosphamide

Cyclophosphamide was administered to the treatment subject by intravenous injection at a dose of 250 mg/m².

### (3) Prednisolone

Prednisolone was administered to the treatment subject at a dose of 2 mg/kg per body weight of the subject intravenously, intramuscularly, subcutaneously, or orally for 7 days, and then at a dose of 1.5 mg/kg in the same manner for 7 days. Thereafter, prednisolone was administered at a dose of 1 mg/kg in the same manner for 7 days, further at a dose of 0.5 mg/kg in the same manner for 7 days, and then the drug was withdrawn.

When the treatment subject was a large dog over 30 kg, prednisolone was administered at a dose of 30 mg/m² intravenously, intramuscularly, subcutaneously, or orally for 7 days, and then at a dose of 20 mg/m² for 7 days in the same manner. Thereafter, prednisolone was administered at a dose of 15 mg/m² in the same manner for 7 days, further at a dose of 10 mg/m² in the same manner for 7 days, and then the drug was withdrawn.

### (4) Doxorubicin

Depending on the body weight of the treatment subject, Adriacin Injection 10 was diluted in 25 to 100 ml of saline to obtain the following initial dose, and then administered intravenously over 30 to 60 minutes.
Initial dose: Treatment subject of less than 10 kg = 1 mg/m²; treatment subject equal to or greater than 10 kg = 30 mg/m²

When doxorubicin could not be used due to the decreased cardiac contractility of the treatment subject, mitoxantrone (trade name: Novantrone) (5 to 6 mg/m²) was used as an alternative.

**[Table 1]**

| No. | Dog Breed | Age at Start of Test | Sex | Contraception / Castration |
|---|---|---|---|---|
| 1 | Belgian Tervuren | 11 years and 10 months old | Female | Done |
| 2 | Hybrid | 5 years and 9 months old | Male | - |
| 3 | Miniature Dachshund | 12 years and 2 months old | Female | Done |
| 4 | Golden Retriever | 13 years and 4 months old | Female | Done |
| 5 | French Bulldog | 3 years and 5 months old | Female | - |
| 6 | Miniature Dachshund | 15 years and 9 months old | Male | Done |
| 7 | Shetland Sheepdog | 12 years and 3 months old | Female | Done |
| 8 | Miniature Schnauzer | 11 years and 5 months old | Male | Done |
| 9 | Miniature Dachshund | 2 years and 7 months old | Male | Done |

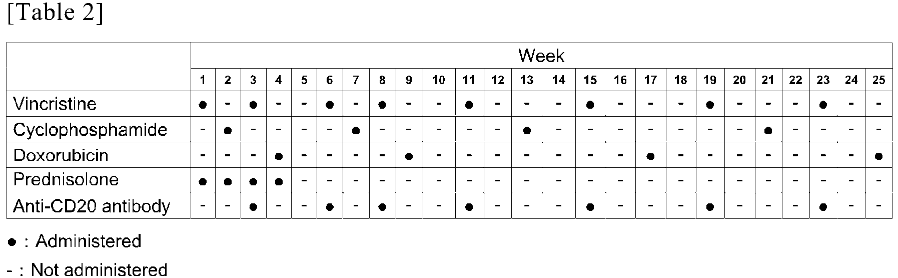

As shown in FIG. 1, it was confirmed that, by the treatment method of the present invention, the relapse of B-cell lymphoma was suppressed even in the period with risk of relapse after completion of the administration (6 to 9 months after the start of administration) in 9 cases of the treatment subjects. Further, in 6 cases out of 8 cases in which 1 year had passed since the start of administration, the relapse was not observed for 1 year or more, and it could be confirmed that survival exceeding 1 year was possible in a plurality of treatment subjects. Furthermore, some of the treatment subjects survived for 2 years or more, or maintained their life for more than two and a half years.

In addition, in these treatment subjects, as a result of starting the treatment method of the present invention, it was confirmed that B cells of the treatment subjects clearly decreased, and the state could be maintained for a long period of time.

Accordingly, from these results, it could be confirmed that the treatment method of the present invention is a method for treating canine B-cell lymphoma having a very high 1-year survival rate and an excellent effect as compared with conventional methods such as the CHOP chemotherapy in which the survival time (median value) after the start of treatment is about 1 year.

As a result of the subsequent follow-up study, it was observed that a plurality of cases survived for two and a half years or more, and some cases survived for more than three and a half years (FIG. 2). In general, the 2-year survival rate of the CHOP chemotherapy is about 20 to 25% (Non-Patent Document 4), but 4 cases out of 10 cases that completed the treatment period survived for 2 years, and thus a remarkable improvement in the survival rate was observed.

### INDUSTRIAL APPLICABILITY

According to the present invention, it is possible to provide a method for treating canine B-cell lymphoma that is more effective than conventional methods. In addition, by providing a kit for treating canine B-cell lymphoma including an anti-canine CD20 monoclonal antibody and a chemotherapeutic agent, it is also possible to treat canine B-cell lymphoma more conveniently.

## Claims

1. A method for treating canine B-cell lymphoma, comprising administering an anti-canine CD20 monoclonal antibody in combination with a chemotherapeutic agent, simultaneously or sequentially,
wherein the antibody comprises a heavy chain variable region and a light chain variable region, wherein the heavy chain variable region comprises V_{H} CDR1 of GFTFNDYWMS (SEQ ID NO: 6), V_{H} CDR2 of DIKYDGSYTNYAPSLKN (SEQ ID NO: 7), and V_{H} CDR3 of EAYYYSGDY (SEQ ID NO: 8), and wherein the light chain variable region comprises V_{L} CDR1 of KTNQNVDYYGNSYMH (SEQ ID NO: 9), V_{L} CDR2 of LASNLAS (SEQ ID NO: 10), and V_{L} CDR3 of QQSRNLPYT (SEQ ID NO: 11).

2. The method according to claim 1, wherein the chemotherapeutic agent is one or more of vincristine, cyclophosphamide, prednisolone, and doxorubicin.

3. The method according to claim 1 or 2, wherein the B-cell lymphoma is canine multicentric large B-cell lymphoma.

4. An anti-canine CD20 monoclonal antibody for use in a method of treating canine B-cell lymphoma,
wherein the method comprises administering said monoclonal antibody in combination with a chemotherapeutic agent, simultaneously or sequentially,
wherein the antibody comprises a heavy chain variable region and a light chain variable region,
wherein the heavy chain variable region comprises V_{H} CDR1 of GFTFNDYWMS (SEQ ID NO: 6), V_{H} CDR2 of DIKYDGSYTNYAPSLKN (SEQ ID NO: 7), and V_{H} CDR3 of EAYYYSGDY (SEQ ID NO: 8), and
wherein the light chain variable region comprises V_{L} CDR1 of KTNQNVDYYGNSYMH (SEQ ID NO: 9), V_{L} CDR2 of LASNLAS (SEQ ID NO: 10), and V_{L} CDR3 of QQSRNLPYT (SEQ **ID** NO: 11).

5. The anti-canine CD20 monoclonal antibody according to claim 4, wherein the chemotherapeutic agent is one or more of vincristine, cyclophosphamide, prednisolone, and doxorubicin.

6. The anti-canine CD20 monoclonal antibody according to claim 4 or 5, wherein the B-cell lymphoma is canine multicentric large B-cell lymphoma.

7. A kit comprising an anti-canine CD20 monoclonal antibody and a chemotherapeutic agent for use in a method of treating canine B-cell lymphoma,
wherein the antibody comprises a heavy chain variable region and a light chain variable region, wherein the heavy chain variable region comprises V_{H} CDR1 of GFTFNDYWMS (SEQ ID NO: 6), V_{H} CDR2 of DIKYDGSYTNYAPSLKN (SEQ ID NO: 7), and V_{H} CDR3 of EAYYYSGDY (SEQ ID NO: 8), and wherein the light chain variable region comprises V_{L} CDR1 of KTNQNVDYYGNSYMH (SEQ ID NO: 9), V_{L} CDR2 of LASNLAS (SEQ ID NO: 10), and V_{L} CDR3 of QQSRNLPYT (SEQ ID NO: 11).

8. The kit according to claim 7, wherein the chemotherapeutic agent is one or more of vincristine, cyclophosphamide, prednisolone, and doxorubicin.

9. The kit according to claim 7 or 8, wherein the B-cell lymphoma is canine multicentric large B-cell lymphoma.
